# EUROPÄISCHE PATENTSCHRIFT

(11) **EP 1 025 828 B1**
(45) Veröffentlichungstag und Bekanntmachung des Hinweises auf die Patenterteilung: **18.09.2002**
(21) Anmeldenummer: 00250031.2
(22) Anmeldetag: 01.02.2000
(51) Int. Cl.: A61H 3/06, G01S 17/02

(54) **Orientierungshilfe für Blinde und Sehbehinderte**
Orientation aid for blinds and visually impaired
Aide d'orientation pour aveugles et mal-voyants

(30) Priorität: 02.02.1999 DE 29902241 U
(43) Veröffentlichungstag der Anmeldung: 09.08.2000
(73) Patentinhaber: VISTAC GmbH, 14513 Teltow (DE)
(72) Erfinder: Ritz, Maria, Dr., 10997 Berlin (DE); König, Lutz, Dr., 10709 Berlin (DE); Wöste, Ludger, Prof. Dr., 14109 Berlin (DE)
(74) Vertreter: Weisse, Renate Dipl.-Phys. Dr.Ing.

(56) Entgegenhaltungen:
- WO-A-98/14149
- DE-A- 19 820 176
- US-A- 3 546 467
- US-A- 3 654 477
- US-A- 4 712 003
- US-A- 5 487 669
- US-A- 5 687 136

## Beschreibung

### Technisches Gebiet

Die Erfindung betrifft ein einen Blindenstock aufweisendes Gerät zur Orientierungshilfe für Blinde und Sehbehinderte nach Anspruch 1.

### Zugrundeliegender Stand der Technik

Blinde oder stark sehbehinderte Personen orientieren sich in ihrer Umgebung in der Regel mit einem Blindenstock, mit welchem Gegenstände in einem Abstand von ca. 1,2 m ertastbar sind. Die Orientierung mittels eines solchen Blindenstock weist mehrere Nachteile auf. Mit dem Blindenstock kann nur Hindernisse in Bodenhöhe und in einem Abstand von ca. 1,2 m ertastet werden. Der Kopf- und Brustbereich der blinden Person bleibt ungeschützt. Hindernisse in dieser Höhe werden oft zu spät erkannt und führen häufig zu Verletzungen.

Es sind eine Mehrzahl von Geräten zur Orientierungshilfe für Blinde und Sehbehinderte bekannt, welche ohne Verwendung eines Blindenstocks diese Nachteile durch Verwendung von berührungslosen Entfernungsmeßsystemen beheben sollen. Solche berührungslosen Entfernungsmeßsysteme sind in verschiedenen Ausführungen bekannt. Dabei wird ein Meßstrahl von einem Sender des Geräts ausgesendet, an einem Gegenstand reflektiert und von einem Empfänger des Geräts empfangen. Daraus wird die Entfernung zwischen dem Gerät und dem Gegenstand entweder durch Laufzeitmessungen oder durch Triangulation ermittelt. Der Meßstrahl kann dabei optisch (z.B. Infrarotstrahl oder Laserstrahl) oder akustisch (z.B. Ultraschall) sein. Die durch das Entfernungsmeßsystem ermittelte Entfernung wird in einem von der Entfernung abhängige Stellgröße umgewandelt, welche einer Anzeigevorrichtung zugeführt wird, durch welche die Entfernung in einer für Blinde oder Sehbehinderte angepaßten Form angezeigt wird. Solche Anzeigen bestehen entweder aus akustischen oder taktilen Signalen.

Ein solches Gerät, welches ohne Verwendung eines Blindenstocks arbeitet, ist in der DE 198 20 176 A1 als Handgerät offenbart. Das Entfemungsmeßsystem ist ein Lasersystem mit einer Laserdiode und einer Photodiode. Die Entfernung wird mittels Laufzeitmessung ermittelt. Dabei sendet die Laserdiode einen Laserstrahl in eine vorgegebene Richtung bzgl. des Handgerätes aus. Die ermittelte Entfernung wird durch eine Anzeigevorrichtung dargestellt, welche eine sich entlang einer Abtaststrecke verlagernde Fühlanzeige enthält.

Ein weiteres solches Gerät, welches ohne Verwendung eines Blindenstocks arbeitet, ist in der US-A-5 487 669 als Handgerät offenbart. Das Entfemungsmeßsystem ist ein Lasersystem. Das Lasersystem enthält eine Sendeeinheit mit einer Laserdiode und eine Empfangseinheit mit einer Photodiode. Die Entfernung wird mittels Triangulation ermittelt. Das Entfernungsmeßsystem ist so ausgelegt, daß es durch das Triangulationsverfahren nicht nur Gegenstände in einem bestimmten Abstand von dem Gerät detektieren kann, sondern in einem ausgedehnten Abstandsintervall. Dies wird durch verschiedene Ausführungen erreicht. Bei einer ersten Ausführung ist die Empfangseinheit so ausgelegt, daß die Photodiode Meßlicht nur aus einer vorgegebenen Richtung empfängt. Der von der Laserdiode ausgesendete Laserstrahl wird durch einen Schwingspiegel periodisch abgelenkt, so daß ein aufgefächertes Laserstrahlbündel entsteht. Die Entfernungsbestimmung erfolgt dann durch Triangulation in Kombination mit einem weiteren Verfahren, bei welchem der Zeitpunkt des Empfangs eines bestimmten Laserpulses in Abhängigkeit von der Position des Schwingspiegels in diesem Zeitpunkt ermittelt wird. Bei einer zweiten Ausführung ist die Sendeeinheit so ausgelegt, daß die Laserdiode Meßlicht nur in eine vorgegebene Richtung aussendet. Die Empfangseinheit ist in diesem Fall so ausgelegt, daß die Photodiode Meßlicht aus mehreren Richtungen empfangen kann. Zu diesem Zweck besteht die Photodiode aus einem CCD mit einer linearen Anordnung von mehreren Dioden. Jede nach Abstand des Gegenstandes, an dem das Meßlicht reflektiert wird, wird das reflektierte Licht von verschiedenen Dioden detektiert. Die Bestimmung der Diode, welche in einem bestimmten Zeitpunkt Meßlicht empfängt, liefert die gesuchte Entfernung. Weiterhin offenbart die US-A-5 487 669 eine dritte Ausführung, bei welcher die Entfernung mittels eines LIDARs durch Laufzeitmessung ermittelt wird, wobei ein Meßstrahl in eine vorgegebene Richtung ausgesendet wird.

Weiterhin sind Blindenstöcke mit Zusatzeinrichtungen bekannt, welche berührungslose Entfemungsmeßsysteme enthalten. Dabei soll das Entfernungsmeßsystem zusammen mit dem Blindenstock Bereiche erfassen, welche mit dem Blindenstock allein nicht erfaßbar sind. Die Strahlenkeule des Entfernungsmeßsystems ist dabei in Gehrichtung ausgerichtet.

Es sind sowohl Zusatzeinrichtungen bekannt, welche einen dreidimensionalen ausgedehnten Raumbereich erfassen, als auch solche, bei welchen der Detektionsbereich sich lediglich in einer vertikal über den Stock liegenden Ebene befindet.

Bei dreidimensionalem Erfassungsbereich sendet die Zusatzeinrichtung eine dreidimensional ausgedehnte Strahlenkeule aus, welche (insbesondere bei Ultraschallgeräten) deutlich über die Reichweite des Blindenstocks hinausgeht. Wenn in diesem Raumbereich ein Hindernis auftritt, dann erhält die blinde Person ein Warnsignal. Es hat sich in der Praxis gezeigt, daß solche bekannte Zusatzeinrichtungen, sei es für sich allein oder in Kombination mit einem Blindenstock, erhebliche Nachteile aufweisen. Es treten Warnsignale auf, welche auf Hindernisse in Entfernungen oder Richtungen schließen lassen, welche für die blinde Person jedoch nicht relevant sind. Die blinde Person wird überhäuft mit Signalen, welche schwer zu interpretieren sind und die blinde Person sogar bei der Orientierung behindert.

Durch die US-A-3 546 467 ist ein Blindenstock mit einer Zusatzeinrichtung bekannt, mit welchem ausschließlich Gegenstände detektiert werden, welche in einer den Blindenstock enthaltenden Ebene liegen. In einem am oberen Teil des Blindenstocks vorgesehenen ersten Gehäuse befinden sich drei Laser, dessen Strahlen in einer gemeinsamen Ebene verlaufen und in drei verschiedene Richtungen ausgestrahlt werden. In einem bestimmten Abstand von dem ersten Gehäuse ist an dem Blindenstock ein zweites Gehäuse mit drei Detektoren vorgesehen, wobei jedem Laser jeweils ein Detektor zugeordnet ist. Das Detektieren von Gegenständen erfolgt mittels Triangulation. Eine erste Laser-Detektor-Einheit erfaßt Gegenstände in einem eindimensionalen Detektionsbereich in Kopfhöhe des Benutzers. Eine zweite Laser-Detektor-Einheit erfaßt Gegenstände in einem eindimensionalen Detektionsbereich in Oberschenkelhöhe des Benutzers. Eine dritte Laser-Detektor-Einheit erfaßt abgehende Stufen in Bodenhöhe ca. ein Meter vor der Stockspitze.

Durch die US-A-3 645 477 ist ein Blindenstock mit einer Zusatzeinrichtung bekannt, mit welchem ausschließlich Gegenstände detektiert werden, welche in einer den Blindenstock enthaltenden Ebene liegen. In einem am oberen Teil des Blindenstocks vorgesehenen Gehäuse befinden sich drei separate Laser-Detektor-Einheiten. Das Detektieren von Gegenständen erfolgt mittels Laufzeitmessung. Eine erste Laser-Detektor-Einheit erfaßt Gegenstände in einem eindimensionalen Detektionsbereich in Kopfhöhe des Benutzers. Eine zweite Laser-Detektor-Einheit erfaßt Gegenstände in einem eindimensionalen Detektionsbereich in Oberschenkelhöhe des Benutzers. Eine dritte Laser-Detektor-Einheit erfaßt abgehende Stufen in Bodenhöhe ca. ein Meter vor der Stockspitze.

### Offenbarung der Erfindung

Der Erfindung liegt die Aufgabe zugrunde, ein einfach aufgebautes Gerät der eingangs genannten Art zu schaffen, durch welches der Kopf- und Brustbereich der blinden Person bei der Orientierung mittels eines Blindenstocks geschützt wird, ohne überflüssige Signale zu erzeugen.

Erfindungsgemäß wird diese Aufgabe durch die im Anspruch 1 angegebenen Merkmale gelöst.

Die Erfindung beruht auf der Erkenntnis, daß ein Gerät zur Orientierungshilfe für Blinde von der blinden Person nur dann als annehmbar akzeptiert wird, wenn die Informationssignale bzgl. ev. Hindernisse ohne aufwendige zeitraubende Überlegungen interpretiert werden können. Dies ist bei den meisten bekannten, mit berührungslosen Meßeinrichtungen arbeitenden Geräten nicht der Fall. Die blinde Person ist an dem bekannten Blindenstock gewöhnt und in der Regel auch im Umgang damit geschult.

Bei der vorliegenden Erfindung wird der Blindenstock nicht durch eine Vorrichtung ersetzt, sondern es handelt sich um eine berührungslos arbeitenden, Detektionsmittel und Anzeigemittel enthaltenden Zusatzeinrichtung, welche in Kombination mit dem normalen Blindenstock eingesetzt wird. Dabei ist die Zusatzeinrichtung so ausgelegt, daß die Funktionsweise des Geräts der Funktionsweise des normalen Blindenstocks ähnlich ist. Durch das erfindungsgemäße Gerät wird nämlich nur ein bestimmter Bereich ("Detektionsbereich") einer bestimmten Ebene ("Detektionsebene") berührungslos abgetastet. Diese Ebene ist so gewählt, daß sie den Blindenstock enthält. Wenn die blinde Person den Blindenstock seitlich hin- und herbewegt, dann wird der Raum oberhalb des Blindenstocks durch die Zusatzeinrichtung erfaßt und zwar so, daß immer nur der jeweilige zweidimensionale Abschnitt des Raums erfaßt wird, der gerade über den Blindenstock liegt. Dabei wird sowohl das Vorhandensein als auch das Nichtvorhandensein eines Gegenstandes in dem Detektionsbereich durch eine bestimmte Information (z.B. durch ein bestimmtes akustisches und/oder taktiles Signal oder durch das Ausbleiben eines bestimmten akustischen und/oder taktilen Signals) angezeigt. Die Wirkung ist dann ähnlich, wie wenn die blinde Person den Blindenstock nicht nur seitlich hin- und herbewegen würde, sondern gleichzeitig auch nach oben und nach unten. (Eine solche Bewegung des Blindenstocks ist natürlich nicht möglich.) Durch diese Funktionsähnlichkeit mit dem normalen Blindenstock erfordert das erfindungsgemäße Gerät keine aufwendige Eingewöhnung oder Umschulung, wie es bei vielen bekannten Orientierungshilfen der Fall ist.

Eine Möglichkeit, die Detektionsmittel so auszulegen, daß sie ausschließlich einen Detektionsbereich in der ausgewählten Detektionsebene abtasten, besteht darin, mehrere von einander unabhängige Laserstrahlen auszusenden, welche alle in der Detektionsebene liegen. Solche Vorrichtungen werden in den oben erwähnten Druckschriften US-A-*3* 546 467 und US-A-3 645 477 beschrieben.

Erfindungsgemäß werden die Detektionsmittel so ausgelegen, daß sie ausschließlich die ausgewählte Detektionsebene abtasten, indem die ausgesandten Laserstrahlen durch Strahlablenkmittel so abgelenkt werden, daß sie in einer Ebene verlaufen. Unter Strahlablenkmittel werden hier Mittel verstanden, welche entweder einen Strahl von einer Richtung in eine andere Richtung umlenkt oder welche einen Strahl so ablenkt, daß die Divergenz des Strahls verändert wird, d.h. daß der Strahl aufgeweitet wird. Diese Strahlablenkung kann beispielsweise durch einen Schwingspiegel erfolgen, welcher beispielsweise Laserstrahlen von einer einzigen Laserquelle periodisch so umlenkt, daß der zweidimensionale Detektionsbereich schrittweise von den umgelenkten Laserstrahlen erfaßt wird. Weitere Strahlablenkmittel wie beispielsweise Prismen können ebenfalls verwendet werden. Die Strahlablenkmittel können jedoch auch Strahlaufweitmittel enthalten, durch welche die ausgesandten Laserstrahlen zweidimensional aufgeweitet werden. Solche Strahlaufweitmittel sind bekannt und können beispielsweise eine Zylinderlinse enthalten. Durch solche Strahlaufweitmittel werden die Laserstrahlen fächerförmig aufgeweitet, wodurch nicht mehrere, nebeneinander verlaufende Laserstrahlen benötigt werden und es auch gewährleistet ist, daß der gesamte ausgewählte Flächenbereich gleichzeitig von der Laserstrahlung erfaßt wird.

Bei dem erfindungsgemäßen Gerät wird zum Detektieren von Gegenständen in der Detektionsebene ein aus Abstandsmessungen bekanntes Meßprinzip verwendet, beispielsweise das Prinzip der Laufzeitmessung, der Triangulationsmessung oder der Phasenmodulationsmessung. Solche Meßprinzipien sind an sich bekannt und werden hier nicht näher beschrieben.

Demgemäß können in einer Ausführungsform der Erfindung die Detektionsmittel zur Laufzeitmessung von Laserstrahlen ausgelegt sein. Dabei sind Laserstrahlerzeugungsmittel zur Erzeugung von Laserpulsen vorgesehen. Weiterhin sind Photodetektormittel vorgesehen, welche auf diese Laserpulse ansprechen, nachdem sie von einem Objekt reflektiert oder gestreut worden sind. Weiterhin sind Signalverarbeitungsmittel vorgesehen, durch welche die Dauer zwischen Aussenden eines Laserpulses durch die Laserstrahlerzeugungsmittel und Detektieren des Laserpulses durch die Photodetektormittel bestimmbar ist. Dabei können die Laserstrahlerzeugungsmittel eine einzige Laserquelle oder eine Vielzahl von Laserquellen und die Photodetektormittel einen einzigen Photodetektor oder eine Vielzahl von Photodetektoren enthalten.

Vorzugsweise sind die Detektionsmittel so ausgelegt, daß der Detektionsbereich der Detektionsebene durch bestimmte Begrenzungen bestimmt ist. Diese Begrenzungen können beispielsweise in Abhängigkeit von der Blindenstocklänge, von dem Blindenstock selbst und von der Kopfhöhe der blinden Person festgelegt sein. Dabei können die Begrenzungen des Detektionsbereichs einstellbar sein. Dadurch können individuell unterschiedliche Kopfhöhen und individuelle Wünsche bzgl. des Abtastbereichs berücksichtigt werden.

Diese Begrenzungen können beispielsweise durch feste oder verstellbare Strahlbegrenzungsmittel zur Begrenzung der ausgesandten Laserstrahlen festgelegt werden. Solche Strahlbegrenzungsmittel können beispielsweise von verwendeten Linsen, z.B. der oben erwähnten Zylinderlinse, gebildet sein. Die Verstellbarkeit kann dabei dadurch gegeben sein, daß verschiedene Zylinderlinsen einsetzbar sind. Als Strahlbegrenzungsmittel können jedoch beispielsweise auch festen oder verstellbare Blenden verwendet werden, welche in den Strahlengang eingesetzt werden.

Weiterhin kann zumindest eine der Begrenzungen durch die Signalverarbeitungsmittel festgelegt werden, wobei ein festes oder verstellbares elektronisches Zeitfenster für das Detektieren des Laserpulses durch die Photodetektormittel definiert wird. Die Größe des Zeitfensters kann beispielsweise über ein Potentiometer elektronisch festgelegt werden. Ein solches Zeitfenster kann dann von einem ausgesandten Laserpuls gesetzt werden. Falls dann die Dauer zwischen Aussenden des Laserpulses und Detektieren des Laserpulses innerhalb des Zeitfensters liegt, dann befindet sich der den Laserpuls reflektierende bzw. zurück streuende Gegenstand innerhalb der Begrenzung des Detektionsbereichs und falls diese Dauer außerhalb des Zeitfensters liegt, dann befindet sich der betreffende Gegenstand außerhalb dieser Begrenzung.

Wenn der Blindenstock mit dem Zusatzgerät gedreht wird, dann wird auch die Detektionsebene mitgedreht. Dies kann bewußt erfolgen, um seitliche Gegenstände abzutasten. Es kann jedoch auch versehentlich erfolgen, wobei die blinde Person dann weiterhin davon ausgeht, daß die detektierte Ebene senkrecht über den Blindenstock liegt. Um dies zu berücksichtigen können Neigungssensormittel zum Feststellen einer Neigung der Detektionsebene gegenüber der senkrechten Ebene vorgesehen sein. Diese Neigungssensormittel können so ausgelegt sein, daß sie ein Signal erzeugen, wenn die Neigung der den Detektionsbereich enthaltenden Detektionsebene gegenüber der senkrechten Ebene einen bestimmten Schwellenwinkel überschreitet. Dieses Signal kann ein von Neigungsanzeigemitteln (z.B. in Form eines Tongebers) ausgelöstes Warnsignal sein, welches die blinde Person darauf aufmerksam macht, daß der Blindenstock bzw. die Detektionsebene falsch ausgerichtet ist. Das Ausmaß der Neigung, bei welchem das Warnsignal ausgelöst wird, wird dabei von dem Schwellenwinkel vorgegeben. Es kann auch vorgesehen sein, eine bestimmte Zeitdauer zwischen Überschreiten des Schwellenwinkels und Auslösen des Warnsignals vorzusehen. Dadurch wird beispielsweise kein Warnsignal ausgelöst, wenn die blinde Person kurzzeitig den Blindenstock dreht, um seitliche Gegenstände abzutasten. Das Signal der Neigungssensormittel kann jedoch auch ein Steuersignal sein, durch welches die Zusatzeinrichtung so gesteuert wird, daß die Detektionsebene gedreht wird, um immer senkrecht zu stehen, oder durch welches der Laser ausgeschaltet wird.

Die Neigungsanzeigemittel können auch aus einem fühlbaren Merkmal an dem Blindenstock bestehen. Beispielsweise kann der Griff asymmetrisch ausgebildet sein, so daß die blinde Person schon beim Anfassen des Blindenstocks feststellen kann, wie dieser ausgerichtet ist.

Die Detektionsmittel, die Anzeigemittel und/oder die Neigungsdetektionsmittel können auf verschiedene Weise an dem Blindenstock untergebracht werden. Beispielsweise können sie in einem gemeinsamen Gehäuse oder in getrennten Gehäusen untergebracht sein, welche in dem Blindenstock integriert oder mittels Befestigungsmitteln an dem Blindenstock befestigbar sein können. In dieser Weise ist es z.B. möglich, bestehende Blindenstöcke mit der Zusatzeinrichtung nachzurüsten.

Ein Ausführungsbeispiel der Erfindung ist nachstehend unter Bezugnahme auf die zugehörigen Zeichnungen näher erläutert.

### Kurze Beschreibung der Zeichnungen

- Fig. 1: ist eine schematische Darstellung und zeigt ein Ausführungsbeispiel eines Geräts zur Orientierungshilfe für Blinde mit einem Blindenstock und eine Zusatzeinrichtung zum Detektieren des Bereichs unmittelbar oberhalb des Blindenstocks.
- Fig. 2: ist eine schematische Blockdarstellung und zeigt die Zusatzeinrichtung von Fig. 1.
- Fig. 3: zeigt den Signalverlauf eines Zeitfensters zur Bestimmung einer Begrenzung des Detektionsbereichs des in Fig. 1 und 2 dargestellten Geräts.

### Bevorzugte Ausführung der Erfindung

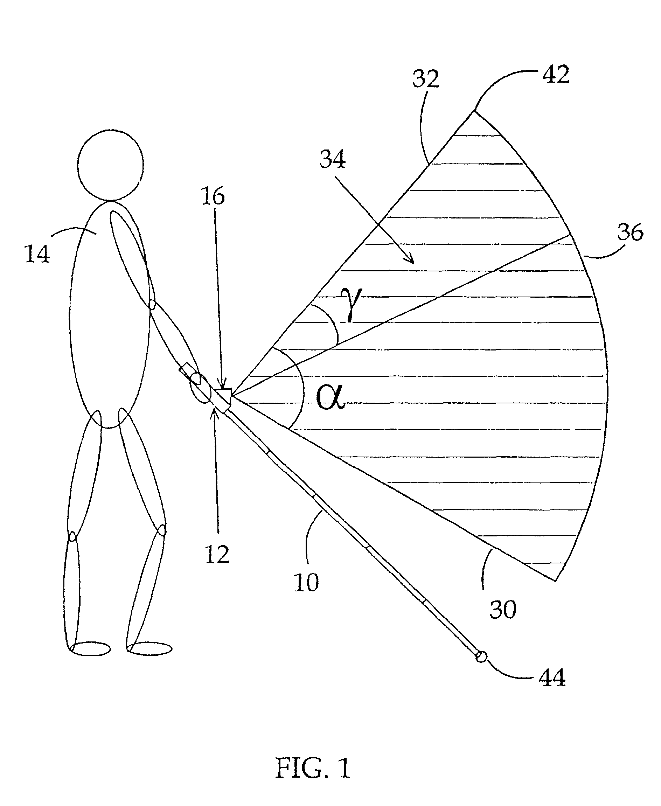

In Fig. 1 ist mit 10 ein Blindenstock mit einem Griff 12 bezeichnet, welcher sich in der Hand einer blinden Person 14 befindet. In der Nähe des Griffs 12 ist eine Zusatzeinrichtung 16 an dem Blindenstock 10 durch geeignete Befestigungsmittel (z.B. durch Schrauben) befestigt.

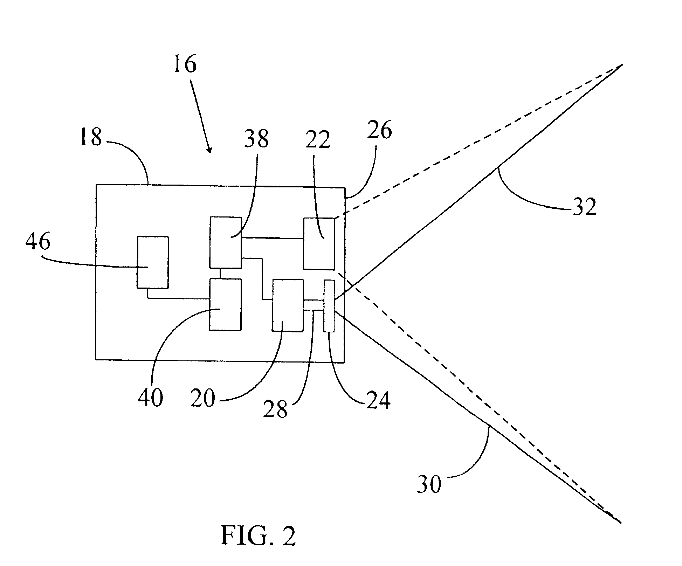

In Fig. 2 ist die Zusatzeinrichtung 16 schematisch dargestellt. Die Zusatzeinrichtung 16 enthält eine in einem mit einer transparenten Frontwand 26 versehenen Gehäuse 18 untergebrachten Lasermeßeinrichtung. Die Lasermeßeinrichtung enthält einen Sender mit einer Laserdiode 20 und einen Empfänger mit einer Photodiode 22. Die hier eingesetzte Lasermeßeinrichtung arbeitet mit Laufzeitmessung zur Abstandsbestimmung. Das Prinzip der Laufzeitmessung beruht darauf, daß die Laserdiode 20 kurze Lichtpulse aussendet und der Empfänger die Zeit zwischen Aussenden des Lichtpulses und Empfang des reflektierten Lichtpulses ermittelt. Eine solche Laufzeit-Lasermeßeinrichtungen ist an sich bekannt und daher hier nicht im einzelnen beschrieben.

Vor der Laserdiode 20 befinden sich l.aserstrahl-Aufweitmittel. In dem dargestellten Ausführungsbeispiel handelt es sich um eine Linienoptik in Form einer Zylinderlinse 24. Der von der Laserdiode 20 ausgehende Laserstrahl 28 wird durch die Zylinderlinse 24 zweidimensional aufgeweitet. Dieser fächerförmig aufgeweitete Laserstrahl ist durch einen ersten und einen zweiten Randstrahl 30 bzw. 32 in Fig. 1 und 2 dargestellt und definiert eine Detektionsebene, welche mit der Papierebene in Fig. 1 und 2 identisch ist. Wie später näher beschreiben werden soll, wird in dieser Detektionsebene ein Detektionsbereich 34 festgelegt. Der Detektionsbereich 34 ist in Fig. 1 schraffiert dargestellt und bildet einen Kreisausschnitt mit drei Begrenzungen, nämlich den ersten Randstrahl 30 als erste Begrenzung, den zweiten Randstrahl 32 als zweite Begrenzung und ein Kreisbogen 36 als dritte Begrenzung.

Der mit der Photodiode 22 ausgestattete Empfänger ist so ausgelegt, daß er die von der Laserdiode 24 ausgesandte und an einem (nicht dargestellten) in dem Detektionsbereich 34 auftretenden Gegenstand reflektierte bzw. gestreute Laserstrahlung empfängt. Zur besseren Darstellung ist der durch gestrichelten Linien dargestellte Empfangsbereich des Empfängers etwas versetzt gegenüber dem durch die Randstrahlen 30 und 32 begrenzten Detektionsbereich 34 dargestellt. Damit Gegenstände in dem gesamten Detektionsbereich erfaßt werden können, muß dabei die Reichweite des Empfängers mindestens so groß sein wie der durch den Detektionsbereich 34 definierte Kreisausschnitt.

Die Photodiode 22 ist mit Signalverarbeitungsmittel 38 verbunden. Weiterhin ist ein Signalgeber 40 mit den Signalverarbeitungsmitteln 38 verbunden. In dem dargestellten Ausführungsbeispiel wird der Signalgeber 40 von einem üblichen Tongenerator gebildet. Wenn der Empfänger einen Gegenstand in dem Detektionsbereich 34 detektiert, wird der Signalgeber 40 von den Signalverarbeitungsmitteln 38 aktiviert und sendet ein Signal aus.

In dem dargestellten Ausführungsbeispiel wird der Detektionsbereich 34 zum einen durch die Auslegung der Laserstrahl-Aufweitmittel (hier Zylinderlinse 24) in Kombination mit der Ausrichtung der Laserdiode 20 und zum anderen durch die Auslegung des Empfängers, insbesondere durch die Auslegung der Signalverarbeitungsmittel 38 des Empfängers bestimmt.

Die Laserstrahl-Aufweitmittel bestimmen den Winkel α des in Fig. 2 dargestellten Kreisausschnitts. Die Lage des Kreisausschnitts bzgl. des Blindenstocks 10 kann durch die Laserstrahl-Aufweitmittel und/oder durch die Ausrichtung der Photodiode 24 bestimmt werden. In dem dargestellten Ausführungsbeispiel wird eine Zylinderlinse 24 gewählt, welche einen Winkel α = 80° liefert. Die Lage des Kreisausschnitts wird durch Ausrichtung der Photodiode 28 und der Zylinderlinse 24 so gewählt, daß bei normaler Führung des Blindenstocks 10 (d.h. schräg nach vorne), der oberste Punkt 42 des Detektionsbereichs 34 sich ca. zwei Meter über den Boden (vorteilhafterweise jedoch mindestens Kopfhöhe der blinden Person) und senkrecht über die Blindenstockspitze 44 befindet. Auf dieser Weise wird nahezu der gesamte Bereich zwischen dem Blindenstock 10 und die Höhe der blinden Person durch den Detektionsbereich 34 abgedeckt.

Die Lage der dritten Begrenzung des Detektionsbereichs 34, nämlich des Kreisbogens 36 des Kreisausschnitts, wird in dem hier dargestellten Ausführungsbeispiel durch die Auslegung der Signalverarbeitungsmittel 38 bestimmt. Dies erfolgt in der Weise, daß durch die Signalverarbeitungsmittel 38 ermittelt wird, ob die von der Photodiode 22 detektierte Laserstrahlung von einem Gegenstand aus einer Entfernung reflektiert bzw. zurück gestreute wurde, welche kleiner als der Abstand zwischen dem Kreisbogen 36 und der Photodiode 22 ist. Dieser Abstand entspricht vorzugsweise der Länge des Blindenstocks 10. Diese Einstellung der Signalverarbeitungsmittel 38 erfolgt durch setzen eines elektronischen Zeitfensters. Dabei werden nur diejenigen reflektierten bzw. zurück gestreuten Lichtpulse berücksichtigt, welche in dem Zeitfenster liegen, d.h. deren Laufzeit ca. der doppelten Länge des Abstands zwischen der Lasermeßeinrichtung und dem Kreisbogen 36 entspricht. Wird innerhalb des Zeitfensters ein Signal von zurück gestreutem oder reflektierten Licht detektiert, dann wird über den Signalgeber 40 ein Warnsignal ausgelöst. Wird außerhalb des Zeitfensters ein Signal von zurück gestreutem oder reflektierten Licht detektiert, dann wird kein Warnsignal ausgelöst.

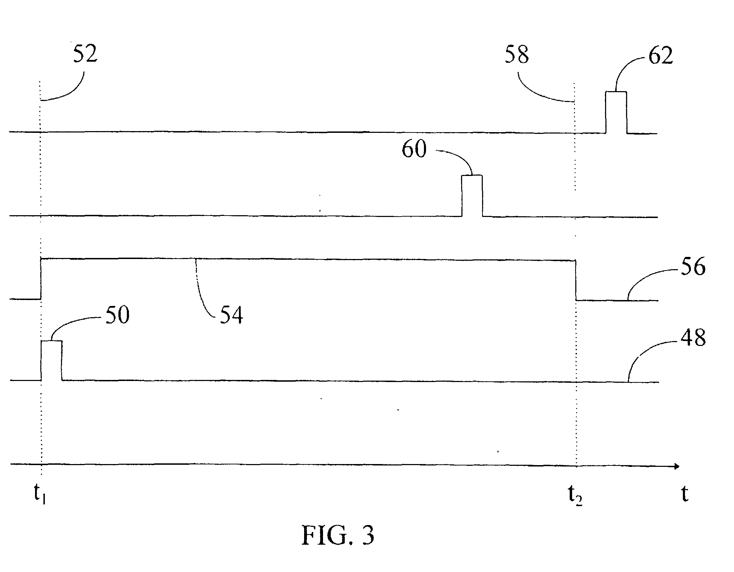

In Fig. 3 ist ein solches Zeitfenster zur Bestimmung der dritten Begrenzung 36 in einem Zeitdiagramm schematisch dargestellt. Ein Pulslasermodul erzeugt ein Monitorsignal, dessen Signalverlauf mit 48 bezeichnet ist. Beim Aussenden eines Laserpulses von der Laserdiode 20 in einem Zeitpunkt t₁ wird das Monitorsignal gesetzt. Dies ist durch einen Signalpuls 50 und eine gestrichelte Linie 52 dargestellt. Dieser Monitorsignalpuls 50 aktiviert eine Signalverzögerungsschaltung der Signalverarbeitungsmittel 38, welche ein Verzögerungssignal 54 erzeugt, dessen Signalverlauf mit 56 bezeichnet ist. Das Ende des Verzögerungssignals 54 ist durch eine gestrichelten Linie 58 angedeutet. Die Dauer t₂ - t₁ des Verzögerungssignals 54 wird beispielsweise durch die Zeitkonstante eines Zeitglieds festgelegt, welche im Falle eines RC-Glieds beispielsweise über ein Potentiometer veränderbar ist. Das Verzögerungssignal 54 wird auf einen Komparator aufgeschaltet. Das Detektorsignal des Photodetektors 22 wird ebenfalls auf den Komparator aufgeschaltet. Der Komparator vergleicht das Verzögerungssignal 54 mit dem Detektorsignal des Photodetektors 22 und stellt fest, ob das Detektorsignal während der Dauer t₂ - t₁ des Verzögerungssignals 54 erscheint oder nicht. Wenn das Detektorsignal durch die Streuung eines Laserpulses der Laserdiode 24 an einem innerhalb der Begrenzung 36 befindlichen Gegenstand ausgelöst wird, dann erscheint das Detektorsignal während dieser Dauer t₂ - t₁ des Verzögerungssignals 54. Ein solches Detektorsignal ist mit 60 bezeichnet. Wenn das Detektorsignal jedoch durch die Streuung eines Laserpulses der Laserdiode 24 an einem außerhalb der Begrenzung 36 befindlichen Gegenstand ausgelöst wird, dann erscheint das Detektorsignal nicht während dieser Dauer t₂ - t₁ des Verzögerungssignals 54. Ein solches Detektorsignal ist mit 62 bezeichnet.

Die Begrenzungen 30, 32 und 36 des Detektionsbereichs 34 bzgl. des Blindenstocks 10 sind verstellbar, z.B. um eine individuell gewünschte Einstellung zu erzeugen. Dies erfolgt einmal durch Änderung des erwähnten Zeitfensters der Signalverarbeitungsmittel 38, wodurch der Abstand zwischen dem Kreisbogen 36 und der Lasermeßeinrichtung verändert wird, und zum anderen durch Änderung des Winkels α (Fig. 1) und die Lage des dargestellten Kreisausschnitts bzgl. des Blindenstocks 10. Zu diesem Zweck können beispielsweise (nicht dargestellte) Blenden vorgesehen werden, welche die Weite des aufgeweiteten Laserstrahls bestimmen. Die Änderung des Winkels α kann aber auch durch Auswechseln der Zylinderlinse 24 erfolgen. In Fig. 1 ist eine alternative Winkelbereich γ von ca. 30° dargestellt, welcher durch Einsatz einer entsprechenden alternativen Zylinderlinse erzeugt wird.

In dem Gehäuse 18 der Zusatzeinrichtung 16 befindet sich weiterhin ein Neigungssensor 46, durch welchen die erforderliche Ausrichtung des Blindenstocks 10 bzw. der Detektionsebene überwacht wird. Solche Neigungssensoren sind an sich bekannt und werden hier nicht näher beschrieben. Mittels eines Regelkreises wird entweder über den Signalgeber 40 oder über einen weiteren Signalgeber ein Warnsignal ausgegeben, wenn die Abweichung der Detektionsebene gegenüber der Senkrechten einen festgelegten Schwellenwinkel (z.B. 15°) überschreitet.

Eine weitere Maßnahme, um eine Neigung der Detektionsebene festzustellen bzw. zu vermeiden, besteht darin, daß der Griff 12 des Blindenstocks 10 asymmetrisch ausgebildet ist. An der geeignet gewählte Asymmetrie kann die blinde Person sofort feststellen, wie der Blindenstock 10 bzw. die Detektionsebene ausgerichtet ist.

In dem Gehäuse 18 befindet sich weiterhin (nicht dargestellte) Stromversorgungsmittel (z.B. Batterien), durch welche die einzelnen Komponenten mit Strom versorgt werden.

Die gesamte Zusatzeinrichtung 16 wird vorzugsweise vollständig in dem Griff 12 integriert oder an dem Griff 12 befestigt. Dies ist aus Gründen der Gewichtsverteilung im Blindenstock 10 sinnvoll.

## Patentansprüche

1. Ein einer Blindenstock (10) aufweisendes Gerät zur Orientierungshilfe für Blinde (14) mit
(a) Detektionsmittel (20, 22, 38), welche durch Aussenden und Empfangen von Laserstrahlen Gegenstände in einem zweidimensionalen Detektionsbereich detektieren, welcher in einer den Blindenstock (10) enthaltenden Detektionsebene liegt, enthaltend
(a₁) Strahlablenkmittel (24) zum Auffächern der ausgesandten Laserstrahlen (28) in diesem Detektionsbereich, und
(a₂) Empangsmittel (22), welche auf die ausgesandten Laserstrahlen ansprechen, nachdem sie von einem in diesem Detektionsbereich befindlichen Objekt reflektiert oder gestreut worden sind,
(b) Anzeigemittel (40), welche
(b₁) das Vorhandensein eines Gegenstandes in dem so erzeugten zweidimensionalen Detektionsbereich (34) durch eine erste Information anzeigen, und
(b₂) das Nichtvorhandensein eines Gegenstandes in dem so erzeugten zweidimensionalen Detektionsbereich (34) durch eine zweite Information anzeigen.

2. Gerät nach Anspruch 1, **dadurch gekennzeichnet, daß** die Detektionsmittel zur Laufzeitmessung von Laserstrahlen ausgelegt sind und
(a) Laserstrahlerzeugungsmittel (20) zur Erzeugung von Laserpulsen aufweisen, sowie
(b) Photodetektormittel (22), welche auf diese Laserpulse ansprechen, und
(c) Signalverarbeitungsmittel (38), durch welche die Dauer zwischen Aussenden eines Laserpulses durch die Laserstrahlerzeugungsmittel (20) und Detektieren des Laserpulses durch die Photodetektormittel (22) bestimmbar ist.

3. Gerät nach Anspruch 1 oder 2, **dadurch gekennzeichnet, daß** die erste und/oder die zweite Information aus akustischen und/oder taktilen Signalen besteht.

4. Gerät nach einem der Ansprüche 1-3, **dadurch gekennzeichnet, daß** die Strahlablenkmittel Strahlaufweitmittel (24) zum zweidimensionalen Aufweiten der ausgesandten Laserstrahlen (28) enthalten.

5. Gerät nach Anspruch 4, **dadurch gekennzeichnet, daß** die Strahlaufweitmittel eine Zylinderlinse (24) enthalten.

6. Gerät nach einem der Ansprüche 1-5, **dadurch gekennzeichnet, daß** die Detektionsmittel (20,22,38) so ausgelegt sind, daß der Detektionsbereich (34) in der Detektionsebene durch bestimmten Begrenzungen (30,32,36) bestimmt ist.

7. Gerät nach Anspruch 6, **dadurch gekennzeichnet, daß** die Begrenzungen (30,32,36) des Detektionsbereichs (34) verstellbar sind.

8. Gerät nach Anspruch 6 oder 7, **dadurch gekennzeichnet, daß** zumindest eine der Begrenzungen (30,32) durch feste oder verstellbare Strahlbegrenzungsmittel (24) zur Begrenzung der ausgesandten Laserstrahlen festgelegt wird.

9. Gerät nach einem der Ansprüche 6-8, **dadurch gekennzeichnet, daß** zumindest eine der Begrenzungen (36) durch die Signalverarbeitungsmittel (38) festgelegt wird, wobei ein festes oder verstellbares elektronisches Zeitfenster (54) für das Detektieren des Laserpulses durch die Photodetektormittel (22) definiert wird.

10. Gerät nach einem der Ansprüche 1-9, **gekennzeichnet durch** Neigungssensormittel (46), welche ein Signal erzeugen, wenn die Neigung der den Detektionsbereich (34) enthaltenden Detektionsebene gegenüber der senkrechten Ebene einen bestimmten Schwellenwinkel überschreitet.

11. Gerät nach einem der Ansprüche 1-10, **gekennzeichnet durch** Neigungsanzeigemittel (40) zum Anzeigen einer Neigung der den Detektionsbereich (34) enthaltenden Detektionsebene gegenüber der senkrechten Ebene.

12. Gerät nach Anspruch 11, **dadurch gekennzeichnet, daß** die Neigungsanzeigemittel ein fühlbares Merkmal an dem Blindenstock (10) enthalten.

13. Gerät nach einem der Ansprüche 1-12, **dadurch gekennzeichnet, daß** die Detektionsmittel (20,22,38), die Anzeigemittel (40) und/oder die Neigungsdetektionsmittel (46) in einem gemeinsamen Gehäuse (18) oder in getrennten Gehäusen untergebracht sind, welche in dem Blindenstock (10) integriert sind oder mittels Befestigungsmitteln an dem Blindenstock (10) befestigbar sind.

14. Gerät nach einem der Ansprüche 1-13, **dadurch gekennzeichnet, daß** die Laserstrahlerzeugungsmittel eine einzige Laserquelle (20) und/oder die Photodetektormittel einen einzigen Photodetektor (22) enthalten.

## Claims

1. A device having a cane (10) to aid the orientation of blind people, having
(a) detection means (20.22.38) which, by emitting and receiving laser beams, detect objects in a two-dimensional detection area located in a detection plane containing the cane (10) and comprise
(a₁) beam deflection means (24) for deflecting the emitted laser beams (28) in this detection area, and
(a₂) receiving means (22) which respond to the emitted laser beams after they have been reflected or scattered by an object located in this detection area,
(b) indicating means (40) which
(b₁) indicate the presence of an object in the thus generated two-dimensional detection area (34) by a first information, and
(b₂) indicate the absence of an object in the thus generated two-dimensional detection area (34) by a second information.

2. Device as set forth in claim 1, **characterized in that** the detection means are adapted for time of flight measurement of laser beams and comprise
(a) laser beam generating means (20) for generating laser pulses,
(b) photodetector means (22) responding to these laser pulses, and
(c) signal processing means (38) arranged to determine the duration between emitting a laser pulse by the laser pulse generating means (20) and detecting the laser pulse by the photodetector means (22).

3. Device as set forth in claim 1 or 2, **characterized in that** the first and/or the second information is represented by acoustic and/or tactile signals.

4. Device as set forth in any one of the claims 1-3, **characterized in that** the beam deflection means comprise beam divergence increasing means (24) for two-dimensionally increasing the divergence of the emitted laser beams (28).

5. Device as set forth in claim 4, **characterized in that** the beam divergence increasing means (24) comprise a cylindrical lens (24).

6. Device as set forth in any one of the claims 1-5, **characterized in that** the detection means (20,22,38) are designed such that the detection area (34) in the detection plane is defined by limits (30,32,36).

7. Device as set forth in claim 6, **characterized in that** the limits (30,32,36) of the detection area (34) are adjustable.

8. Device as set forth in claim 6 or 7, **characterized in that** at least one of the limits (30,32,36) is defined by fixed or variable beam limiting means (24) for limiting the emitted laser beams.

9. Device as set forth in any one of the claims 6-8, **characterized in that** at least one of the limits (36) is defined by the signal processing means (38), a fixed or variable electronic time window (54) for detecting the laser pulse by the photodetector means (22).

10. Device as set forth in any one of the claims 1-9, **characterized by** inclination sensor means (46) which generate a signal when the detection plane containing the detection area (34) is inclined by more than a predetermined threshold angle relative to the vertical plane.

11. Device as set forth in any one of the claims 1-10, **characterized by** inclination indicating means (40) for indicating an inclination of the detection plane containing the detection area (34) relative to the vertical plane.

12. Device as set forth in claim 11, **characterized in that** the inclination indicating means comprise a perceptible feature provided on the cane (10).

13. Device as set forth in any one of the claims 1-12, **characterized in that** the detection means (20,22,38), the indicating means (40) and/or the inclination sensor means (46) are accommodated in a common housing (18) or in separate housings which are integrated in the cane (10) or adapted to be attached to the cane (10) by means of attachment means.

14. Device as set forth in any one of the claims 1-13, **characterized in that** the laser beam generating means comprise one single laser source (20) and/or the photodetector means comprise one single photodetector (22).

## Revendications

1. Instrument présentant une canne pour non-voyants (10), destiné à aider les non-voyants (14) à s'orienter et muni de
(a) moyens de détection (20,22,38) qui détectent des objets en émettant et en recevant des rayons laser dans un domaine de détection bidimensionnel se trouvant dans un plan de détection comprenant la canne pour non-voyants (10); comprenant,
(a₁) des moyens de déviation de rayons (24) destinés à disperser en éventail les rayons laser émis (28) dans ce domaine de détection, et
(a₂) des moyens de réception (22) répondant aux rayons laser émis après leur réflexion ou leur dispersion par un objet se trouvant dans ce domaine de détection,
(b) moyens d'affichage (40) affichant
(b₁) la présence d'un objet dans le domaine de détection bidimensionnel (34) ainsi généré par une première information, et
(b₂) l'absence d'objet dans le domaine de détection bidimensionnel (34) ainsi généré par une seconde information.

2. Instrument selon la revendication 1, **caractérisé par le fait que** les moyens de détection sont dimensionnés pour la mesure du temps de parcours de rayons laser et
(a) qu'ils présentent des moyens de génération de rayons laser (20) destinés à générer des impulsions laser ainsi que
(b) des moyens de photodétecteur (22) répondant à ces impulsions laser, et
(c) des moyens de traitement de signaux (38) grâce auxquels la durée entre l'émission d'une impulsion laser par les moyens de génération de rayons laser et la détection de l'impulsion laser par les moyens de photodétecteur (22) est susceptible d'être déterminée.

3. Instrument selon la revendication 1 ou 2, **caractérisé par le fait que** la première et/ou la seconde information se compose de signaux acoustiques et/ou tactiles.

4. Instrument selon l'une des revendications 1-3, **caractérisé par le fait que** les moyens de déviation de rayons comprennent des moyens d'élargissement de rayons (24) destinés à élargir bidimensionnellement les rayons laser émis (28).

5. Instrument selon la revendication 4, **caractérisé par le fait que** les moyens d'élargissement de rayons comprennent une lentille cylindrique (24).

6. Instrument selon l'une des revendications 1-5, **caractérisé par le fait que** les moyens de détection (20,22,38) sont dimensionnés de telle façon que le domaine de détection (34) est déterminé dans le plan de détection par certaines délimitations (30,32,36).

7. Instrument selon la revendication 6, **caractérisé par le fait que** les délimitations (30,32,36) du domaine de détection (34) sont susceptibles d'être déplacées.

8. Instrument selon la revendication 6 ou 7, **caractérisé par le fait que** au moins l'une des délimitations (30,32) est fixée par des moyens de délimitation de rayons fixes ou susceptibles d'être déplacés (24) destinés à délimiter les rayons laser émis.

9. Instrument selon l'une des revendications 6-8, **caractérisé par le fait que** au moins l'une des délimitations (36) est fixée par les moyens de traitement de signaux (38), un créneau horaire (54) électronique fixe ou susceptible d'être modifié pour la détection de l'impulsion laser est défini par les moyens de photodétecteur (22).

10. Instrument selon l'une des revendications 1-9, **caractérisé par** des moyens de capteur d'inclinaison (46) générant un signal quand l'inclinaison du plan de détection comprenant le domaine de détection (34) par rapport au plan vertical dépasse un certain angle de seuil.

11. Instrument selon l'une des revendications 1-10, **caractérisé par** des moyens d'affichage de l'inclinaison (40) destinés à afficher une inclinaison du plan de détection comprenant le domaine de détection (34) par rapport au plan vertical.

12. Instrument selon la revendication 11, **caractérisé par le fait que** les moyens d'affichage de l'inclinaison comprennent une caractéristique tactile sur la canne pour non-voyants (10).

13. Instrument selon l'une des revendications 1-12, **caractérisé par le fait que** les moyens de détection (20,22,38), les moyens d'affichage (40) et/ou les moyens de détection de l'inclinaison (46) sont logés dans un boîtier commun (18) ou dans des boîtiers séparés qui sont intégrés dans la canne pour non-voyants (10) ou qui sont susceptibles d'être fixés sur la canne pour non-voyants (10) au moyen de moyens de fixation.

14. Instrument selon l'une des revendications 1-13, **caractérisé par le fait que** les moyens de génération de rayons laser comprennent une seule source laser (20) et/ou les moyens de photodétecteur comprennent un seul photodétecteur (22).
